# EUROPEAN PATENT APPLICATION

(11) **EP 1 382 347 A1**
(43) Date of publication of application: **21.01.2004**
(21) Application number: 02291811.4
(22) Date of filing: 17.07.2002
(51) Int. Cl.: A61K 38/18, G01N 33/50, A61P 21/00, A61P 25/00

(54) **Use of CCN protein family members for the treatment of disorder associated to an altered calcium and/or sodium flux**

(71) Applicant: UNIVERSITE PARIS 7 - Denis DIDEROT, F-75005 Paris (FR)
(72) Inventor: Perbal, Bernard, 78280 Guyancourt (FR)
(74) Representative: Warcoin, Jacques

(57) **Abstract**

The invention relates to the use of a CCN protein for the preparation of a drug for the treatment of a disorder associated to an altered calcium and/or sodium flux, and the pharmaceutical composition thereof.

## Description

The invention relates to a pharmaceutical composition for the preparation of a drug against a disease associated to a disorder in calcium and/or sodium flux.

More specifically, the invention relates to compositions comprising at least one CCN protein, and in particular NOV protein (Nephroblastoma overexpressed protein), efficient in calcium and/or sodium transfer control.

Numerous drugs intend to act on the control of calcium and/or sodium. Indeed calcium and sodium are involved in many biological mechanisms.

Intracellular calcium fluxes are controlled very strictly by proteins that link calcium, and by receptors that allow a precise dosage of the calcium quantities in the different cellular compartments. The regulation of the expression of numerous genes by calcium has been studied for a long time. The functioning of neurons and synaptic activity are heavily dependent on calcium ions (Ca²⁺). For example, calcium is involved in LTP , in the regulation of membrane excitability and serves directly as a second and third messenger in neurons. Moreover, several lines of evidence from rodent species point to the hypothesis that changes in the neuronal calcium homeostasis coincide with aging of the brain in general, and may be correlated with age-related decline in cognitive functions. For example, hippocampal calcium channels increase their activity in aged brain and the density of L-type calcium channels is increased in aged hippocampal CA1 neurons. The experimental evidence has led to the suggestion that changed calcium homeostasis in aged neurons may be a contributing factor to some memory deficits caused by aging. Besides, high voltage activated calcium currents were enhanced in dentate granule neurons from aged rats by EGTA introduced intracellulary in the recording electrode. Also, reduced field Excitatory Postsynaptic Potential (fEPSP) in the hippocampal slices of old rats relative to that observed in the younger age groups was found to be correlated with impaired spatial learning in aged animals. Further evidence supporting the hypothesis that a calcium increase in aged neurons may be implicated in memory impairment comes from in vivo studies on the role of the calcium channel blocker, nimodipine. This drug reduced the number of age-related motor impairments in aged rats, and enhanced their recent memory. There is still a need of drugs efficient to control calcium flux.

Concerning sodium it is also known in the art that a deficient control of sodium flux leads to different disorders. The positive action of sodium channel blockers is for instance described in patent US 6 414 011, for treating or preventing neuronal loss due to focal or global ischemia, for treating or preventing neurodegenerative disorders including ALS, anxiety, and epilepsy. They are also expected to be effective in treating, preventing or ameliorating neuropathic pain, surgical pain, chronic pain and tinnitus. The compounds are also expected to be useful as antiarrhythmics, anesthetics and antimanic depressants. Thus there is still a need of drugs efficient to control sodium flux.

The inventor has now demonstrated the role of certain CCN proteins, in particular of the NOV protein, in the control of calcium and of sodium flux. Besides, considering these results and the data relating calcium and/or sodium transport to different disorders, there is now a strong support to the use of CCN proteins, namely of NOV protein (CCN3), for the prophylaxis or treatment of these disorders.

The NOV protein also named CCN3 is a member of the CCN [CYR61 (cystein rich), CTGF (connective tissue growth factor) and NOV (nephroblastoma overexpressed) family of regulatory proteins (Bork, 1993 ; Lau *et al.,* 1999 ; Perbal, 2001). With Elml, rCOP1 and WISP (wint induced secreted proteins) (Hashimoto *et al.,* 1998 ; Zhang *et al.,* 1998 ; Pennica *et al,* 1998) the CCN family now consists of six members (the nomenclature used by the inventor is issued from the International CCN Society (http://CCNsociety.jussieu.fr), and refers to NOV, CTGF, CYR61, WISP1, WISP2, WISP3 nomenclatures currently used for human.). The NOV (CCN3) gene was originally described as a target for MAV (Myeloblastosis Associated Virus) in avian nephroblastomas (Joliot et al., 1992) which represent a unique model of the Wilms tumour (Perbal, 1994). Although its expression was found to be greater in all avian tumors than in normal kidneys, NOV (CCN3) was only disrupted in one nephroblastoma and insertional activation of MAV in the vicinity of NOV (CCN3) is not a common theme in nephroblastoma *(Li et al. Manuscript in preparation).*

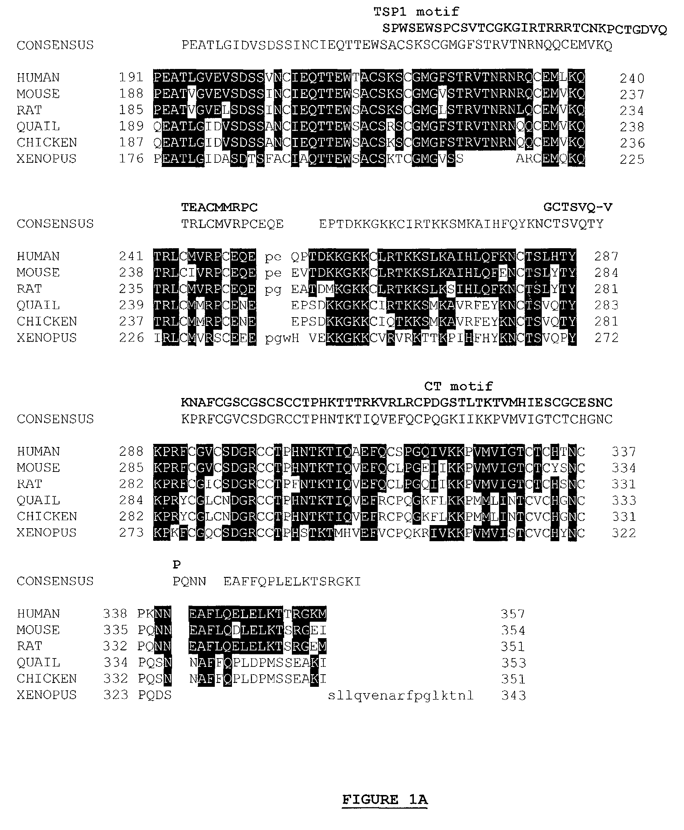

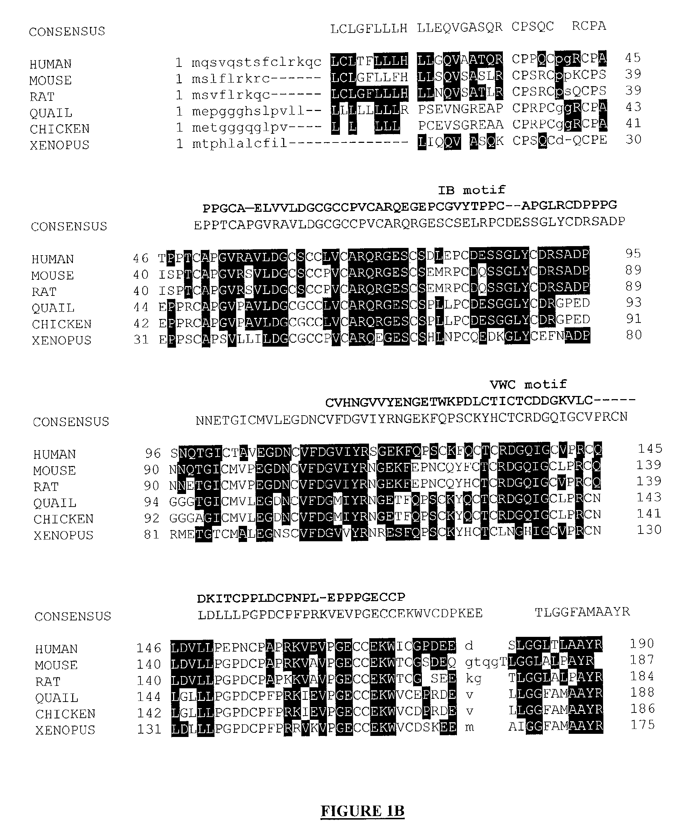

As other members of the CCN family, the NOV (CCN3) protein shows some striking features. It is composed of five distinct structural modules encoded by separate exons and contains a total of 38 cystein residues, whose position in the four modules was conserved both throughout evolution and among the different members of the CCN family (Perbal, 2002). The structural conservation of NOV (CCN3) in the different species where it has been identified is suggesting that this protein is fullfilling important function(s) that are dictated by a common spatial organisation. It is noteworthy that in the first two modules of NOV (CCN3), a total of eleven proline residues are localized at the same position from xenopus to human (figure 1). The presence of a signal peptide at the aminoterminus of the CCN proteins is responsible for the secretion of the full length NOV (CCN3) protein which can be released in the extracellular matrix or remain at the cell membrane (Perbal, 1994 ; Thomopoulos *et al.,* 2001). There is an increasing number of examples reporting the existence of CCN isoforms lacking one or two of the basic modules. The amino truncation of NOV (CCN3) resulting from MAV insertion in target cell DNA conferred on the NOV (CCN3) deprived of signal peptide and module 1 (IB, sharing partial identity with IGFBPs), a transforming potential (Joliot *et al.,* 1992) and high levels of an aminotruncated NOV (CCN3) isoform, deprived of modules 1 and 2 (VWC sharing identity with the type C repeat of Von Willebrand factor), are detected in the nucleus of cancer cells (Perbal, 1999). Module 4 (CT, sharing identity with growth factors) is not present in CCN5 (rCOP/WISP2 and CTGFL) (Hashimoto et al., 1998 ; Pennica *et al.,* 1998 ; Kumar *et al.,* 1999), and module 2, is absent in WISP1v, a short version of CCN4(WISP1) expressed in gastric carcinoma (Tanaka *et al.,* 2001). These observations raised interesting questions as to the biological functions of these different isoforms and the mechanisms by which they are generated.

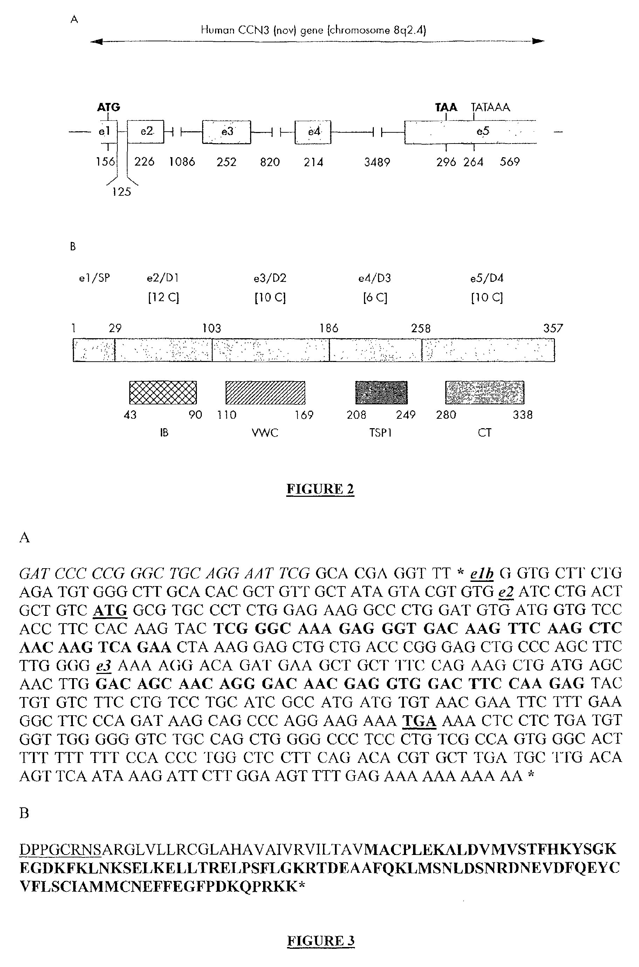

The nov gene (ccn3 gene) spans about 7,5 kb of DNA (figure 2) and maps on human chromosome 8q24 (Martinerie *et al.,* 1992). Its expression in normal conditions is submitted to a tight spatial and temporal regulation. The expression of NOV (CCN3) during normal development has been associated both in human, rodents and birds to the polarized differentiation of different cell types in various systems including cartilage and bone, central nervous system, kidney, striated and cardiac muscle (Perbal, 2001 ; Chevalier *et al.,* 1998 ; Kocialkowski *et al.,* 2001 ; Su By *et al.,* 1998 ; Katsube *et al.,* 2002). The major sites of NOV (CCN3) expression in human, rodent and chicken were identified by *in situ* hybridization and immunocytochemistry as *zona fasciculata* of the adrenal, neurons and astrocytes in the nervous system, ganglia and fusing muscle cells (Perbal, 2001 ; Chevalier *et al.,* 1998 ; Kocialkowski *et al.,* 2001 ; Su By *et al.,* 1998 ; Katsube *et al.,* 2002). In pathological conditions, the expression of both human NOV (CCN3) mRNA species and NOV (CCN3) protein was shown to be impaired in many different types of tumours (See Perbal, 2001 for a review). In the case of neuroblastomas (our unpublished results), Wilms tumours (Chevalier *et al.,* 1998), osteosarcomas and chondroblastomas (Manara *et al.,* 2002 ; Ayer-Lelievre et al., 2001), the expression of NOV (CCN3) was marker of differentiation, whereas in the case of renal cell carcinomas (Glukhova *et al.,* 2001), prostate carcinomas (Maillard *et al.,* 2001), and Ewings tumours (Manara *et al.,* 2002), expression of NOV (CCN3) was associated with increased cellular proliferation.

As far as calcium flux is concerned, the inventor has now shown the action of NOV (CCN3) protein on the calcium flux. Before the present invention, protein NOV (CCN3) was shown to be found in numerous tissues in which calcium fluxes play an important role, namely : the adrenal gland, the cardiomyocytes, the glomerules and renal tubules, the central nervous system, the skeletal muscles, the regions of bone growth. However, there was still a need for a good understanding of the mechanism of action of CCN and particularly of NOV (CCN3) proteins, and there was no evidence of implication of NOV (CCN3) protein in calcium flux control. It has to be mentioned that in such a complex biological field, there is a long way between having an idea of the action of a protein on a biological mechanism, and the actual proof of such action. The obtention of such proof needs a significant inventive step and to solve many technical problems.

More specifically, the inventor has now shown that the NOV protein also named CCN3, physically interacts with the calcium binding protein S100A4 and induces a significant transient increase of intercellular calcium in glioblastoma and nervous system cells, clearly giving evidence to the fact that NOV (CCN3) has a pivotal role in calcium dependent signalling.

Thus the invention relates according to an aspect to the use of a CCN protein for the preparation of a drug for the treatment of a disorder associated to an altered calcium flux. The CCN protein may be any of the above mentioned proteins : NOV, CTGF, CYR61, WISP1, WISP2, WISP3, and their natural variants thereof (polymorphism) and in particular NOV protein more preferably NOVH. According to the present invention the term NOV protein is similar to CCN3 protein (and may be equally used) ; this term encompasses human NOV protein (NOVH), but also any animal NOV protein, and also all the variants (natural polymorphism), chimeric or fusion NOV proteins (such as GST-NOV) that display the biological function of the native NOV protein. Further, considering the structural conservation between CCN proteins (medicine/sciences, Perbal, 2002 n°6-7, vol 18, pages 745-756), there is a strong support for the analogous properties of other CCN proteins.

For calcium, the disorder is namely chosen among the main following categories : cellular repair, signalisation and cellular adhesion mediated by integrins, cellular interaction and communication, dysfunction of cardiac cells and/or skeletal muscles cells (in particular pathology of muscular contraction, neurodegenerative disease), altered bone growth or bone repair.
The basis of such therapeutic effect is namely as follows.
- Cellular repair :
   The intracellular calcium concentration increases within the few seconds following cellular injuries. The intracellular calcium contributes also to the signalling of the cellular adhesion mediated by the integrins. The role of integrins in the control of intercellular adhesion and the interaction with the extra-cellular matrix is also known. The fixing of calcium is essential for optimal activity of integrins.
- Cellular interactions and communications using a Gap-junction :
   The intercellular calcium fluxes are important for the coordination of cellular activities and the proper metabolic function of tissues. These interactions are very important for the function of the nervous system. The inventor and coworkers had shown that NOV (CCN3) interacts with the connexine 43 at the level of Gap-junctions of glioblastoma cells (Gupta *et al.,* 2001).
- Cardiac activity and activity of muscles :
   The delivery of calcium by sarcoplasmic reticulum is a key step linking the membranar depolarisation, and the mechanical activity during the successive phases of excitation and contraction in the cardiac cells and the cells of the skeletic muscles. The control of the intracellular calcium level by NOV (CCN3) implies that this protein might be useful in the cardiac pathologies associated to altered calcic fluxes. The pathologies of muscular contraction and neurodegenerative diseases are also probable applications.

Further, the inhibition of myoplastic differentiation by NOV (CCN3) protein implies that this NOV can maintain the muscular precursors in culture and allow reconstituting the muscular tissues by gene therapy and stem cells use.
- Bone growth :
   NOV (CCN3) protein is known to be associated to the terminal differentiation of chondrocytes. It is highly probable that NOV (CCN3) is involved during the transition towards bone formation, phases during which the calcium flux is essential. Thus, NOV (CCN3) would be very useful for repairing broken bones. Further, during bone reconstitution often necessary during teeth implantation, the best process of the prior art involves the use of a mixture of calcic phosphates and of platelets issued from the patient, in order to stimulate bone neo-formation. It is known that the integrins are associated to blood platelets and that protein CCN, with which they interact, are also expressed in platelets. Such a reconstitution process usually needs several months. Thus, adding of CCN proteins would be of great help for reducing this delay. Further, the inventor has shown that glioblastoma cells which express NOV (CCN3) are less tumorigenic than the sister cells which do not express NOV (CCN3). Thus NOV (CCN3) proteins are likely to have anti-tumoural action. NOV (CCN3) injection will be helpful for the treatment of tumours of the nervous central system.

As far as sodium flux is concerned, the inventor has now demonstrated the blocking action of NOV (CCN3) protein on sodium flux, showing that this protein can be used in numerous situations normal or pathological, in order to ameliorate nervous influx propagation. Thus the invention also relates to the use of NOV (CCN3) protein for the preparation of a medicament against a disorder associated to altered sodium flux.

For sodium, the action of NOV (CCN3) protein or other CCN proteins may be used mainly in the following fields :
- Antalgic and analgesic activity (anti pain, local anaesthesia, desensibilisation at temperatures...)
- Inhibition of nervous conduction by a local application of the protein (which is stable in culture medium) for the treatment of behaviour disorder.
- Treatment of pathologies of central nervous system due to altered nervous conduction, without undesired side effects in view of the reversible effect of NOV (CCN3) protein.

Further, the fact that the NOV (CCN3) compound is a protein allows further uses in gene therapy of neuromuscular diseases for instance, or other pathologies of nervous transmission.

Besides, the inventor has shown the expression of NOV (CCN3) also in regions of the brain associated to sensitive functions such as listening and knowing functions (learning and memory). Ectopic use of NOV (CCN3) is then also possible. Considering that, as it will be described, NOV (CCN3) protein allows to obtain similar results as tetrodoxin which is known as sodium channel blocking compound, there is a strong support on the applications just mentioned.

The inventor reminds examples of therapeutic uses of tetrodoxin mentioned in US patent N° 6,407,088. Tetrodoxin compounds, namely tetrodotoxin, anhydrotetrodotoxin, tetrodaminotoxin, methoxytetrodotoxin, ethoxytetrodotoxin, deoxytetrodotoxin and tetrodonic acid are as sodium channel blocking compound known to produce analgesia in a mammal. Their systemic administration is performed by intramuscular injection, subcutaneous injection, intravenous injection, oral ingestion, sublingual ingestion, skin patch, implantable osmotic pump, collagen implant, aerosol inhalation, or suppository.

The pain may be caused namely by mechanical, chemical or ischemic stimulation, or inflammation. The pain may be for instance a neuropathic pain, or arise from cancer selected from the group consisting of liver cancer, rectal cancer, leiomyosarcoma, bone cancer, stomach cancer, lymphatic cancer, esophageal cancer, cancers in the genital organs, cancers of endocrine glands, prostate cancer, digestive system cancer, stomach cancer, colon cancer, breast cancer, respiratory system cancer, lung cancer, bronchial cancer, urinary system cancer, lymphoma and skin cancer. Such sodium channel blocking compounds are administered typically in a dose of 0.1 to 5 µg per kilogram body weight, in a dosage range for example in a schedule of up to 4 doses per day over a time period of 3 days. Frequently the effectiveness of the dose lasts for up to 20 days.
Adams *et al.,* (U.S. Pat. Nos. 4,022,899 and 4,029,793) pertain to a local anesthetic composition comprising a mixture in a pharmaceutically acceptable carrier of a namely tetrodotoxin or desoxytetrodotoxin, and another compound, generally a conventional local anesthetic compound or a similar compound having nerve-blocking properties. Pan *et al.,* (U.S. Pat. No. 5,846,975) discloses the use of amino-hydrogenated quinazoline compounds, such as tetrodotoxin, for treating drug dependence in humans. Tetrodotoxin was shown to be effective against withdrawal symptoms from opium, heroin, morphine, cocaine, amphetamine, dolandin, dihydroetorphine and methadone. Amounts effective for relieving withdrawal symptoms are described in this patent. Tetrodotoxin can be used as a local anesthetic and is ten thousand times more powerful than commonly used local non-narcotics, as is discussed by C. Y. Kao and F. A. Fuhrman (J. Pharmacol., 140, 31-40 ; 1963). Tetrodotoxin preparations in combination with other widely used anesthetics have been noted in U.S. Pat. Nos. 4,022,899 and 4,029,793.

U.S. Pat. No. 6,030,974 describes a method of producing local anaesthesia in a mammal experiencing pain in an epithelial tissue region. The method includes topically administering to the region, in a suitable pharmaceutical vehicle, an effective dose of a long-acting sodium channel blocking compound.

The sodium channel blocking compound of U.S. Pat. No. 6,030,974 can be a formulation of tetrodotoxin or saxitoxin at a concentration of between 0.001-10 mM. Several other sodium channel blockers such as lidocaine and carbamazepine have been used in the treatment of neuropathic pain and trigeminal neuralgia.

The invention also relates to a pharmaceutical composition comprising at least one CCN protein, namely NOV (CCN3) protein, and a pharmaceutically acceptable carrier. The composition may be active only for sodium flux control, only for calcium flux control, or both calcium and sodium flux according to the disorder of the patient. The composition may comprise a CCN protein and another sodium channel blocker for a synergic effect. Such compositions can be screened using the measurement protocol used for NOV (CCN3) described below.

When used therapeutically, the compound of the invention is administered in therapeutically effective amount. In general, a therapeutically effective amount means that amount necessary to delay the onset of, inhibit the progression of, or halt altogether the particular disorder being treated. Therapeutically effective amounts specifically will be those which desirably influence the calcium and/or sodium influx in the target cells. Obviously, if appropriate, the clinician will take care not to use the compound in case of a disorder due to an excess in neuronal cell calcium influx for example described in US 6,172,043.

Generally, a therapeutically effective amount will vary with the subject's age, and condition, as well as the nature and extent of the disease in the subject, all of which can be determined by one of ordinary skill in the art. The dosage may be adjusted by the individual physician, particularly in the event of any complication.
A therapeutically effective amount typically varies from 0.01 mg/kg to about 1000 mg/kg, preferably from about 0.1 mg/kg to about 200 mg/kg and most preferably from about 0.2 mg/kg to about 20 mg/kg, in one or more dose administrations daily, for one or more days.

The administration of a CCN protein can be adjusted for instance by comparison with the dosage of other proteins used in the disorders mentioned above and of tetrodoxin which is nonproteic neurotoxin reminded below.

| Administration of Tetrodotoxin. | | |
|---|---|---|
| Route of Administration | Dose (µg/50 kg subject) | Schedule |
| Intramuscular injection | 5-50 | 4 .about. 2/day |
| Intravenous injection | 5-30 | 3 .about. 2/day |
| Subcutaneous injection | 5-50 | 4 .about. 2/day |
| Sublingual | 5-30 | 3 .about. 2/day |
| Patch through skin | 5-60 | 4 .about. 2/day |
| Oral ingestion | 5-30 | 3 .about. 2/day |
| Implantable Osmotic pump | 30-60 | 1 |
| Collagen implants | 30-60 | 1 |
| Aerosol | 5-50 | 4 .about. 2/day |
| Suppository | 5-30 | 3 .about. 2/day |

Formulation for each administration route in Table 1 is generally considered known in the art. [See, e.g., "Remington, the Science and Practice of Pharmacy", 19^{th} ed., A. R. Gennaro, ed., 1995 by The Philadelphia College of Pharmacy and Science, (especially Part 7)]. As shown in Table 1, the typical dose ranges from 5 to 60 µg per adult. A more typical dose is from 20 to 40 µg per adult.

The therapeutics of the invention can be administered by any conventional route, including injection or by gradual infusion over time. The administration may, for example, be oral, intravenous, intracranial, intraperitoneal, intramuscular, intracavity, intrarespiratory, subcutaneous, or transdermal. The route of administration will depend on the composition of a particular therapeutic preparation of the invention. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Other delivery systems can include time-release, delayed release or sustained release delivery systems. Such systems can avoid repeated administrations of the active compounds of the invention, increasing convenience to the subject and the physician. Many types of release delivery systems are available and known to those of ordinary skill in the art. They include polymer based systems such as polylactic and polyglycolic acid, polyanhydrides and polycaprolactone; nonpolymer systems that are lipids including sterols such as cholesterol, cholesterol esters and fatty acids or neutral fats such as mono-, di and triglycerides; hydrogel release systems; silastic systems; peptide based systems; wax coatings, compressed tablets using conventional binders and excipients, partially fused implants and the like. In addition, a pump-based hardware delivery system can be used, some of which are adapted for implantation. A long-term sustained release implant also may be used. "Long-term" release, as used herein, means that the implant is constructed and arranged to deliver therapeutic levels of the active ingredient for at least 30 days, and preferably 60 days. Long-term sustained release implants are well known to those of ordinary skill in the art and include some of the release systems described above.

It is here reminded that sodium channel blockers are known to be effective as long-term systemic analgesics for alleviation of severe pain.

The invention also relates to a method for the treatment and/or prophylaxis of a disorder associated to altered calcium and/or sodium flux which comprises administering to a patient in need of such treatment an effective amount of at least a CCN protein, namely NOV(CCN3) protein.

The invention also relates to a method for administering NOV (CCN3) protein to a mammal so as to sustain its biological response in the treatment of a chronic disorder in the mammal comprising administering a therapeutically effective amount NOV (CCN3) protein. As used herein, "biological response" refers to the favourable response of a mammal having a specific chronic disorder to treatment with NOV (CCN3), maximizing the response means improving efficacy but at the same time avoiding or at least minimizing the occurrence of side effects. As used herein "chronic" refers to a disorder that is not acute but rather occurs more or less on a continuous level. A "disorder" is any condition that would benefit from treatment with CCN in particular NOV (CCN3) protein.

According to a further aspect the invention relates to a method of screening of a CCN protein efficient against a disorder associated to altered calcium and/or flux, comprising adding an appropriate dosage of a tested CCN protein, and measuring the intracellular calcium level, preferably in comparison with a control efficient CCN protein, preferably NOV (CCN3) protein. The protocol for measuring the a ctivity may be the one used in the present application and further described in detail.

The invention also relates to the use of a compound obtained by the screening method above for the treatment and/or prophylaxis of a disorder associated to altered calcium and/or sodium flux. The present application, considering the protocols described in detail and the high conservation between the CCN proteins, gives sufficient data for the obtention of active candidates and their therapeutic use without undue experiment.

Further objects and advantages of the invention will be further apparent from the following detailed description, illustrated by the drawings :

### Concerning calcium flux :

- Figure 1a : Amino acid sequence alignment in NOV (CCN3) proteins. The amino acid sequence of NOV (CCN3) proteins from different species has been aligned with the interalign program (University of Sandford, USA). Those residues whose position is conserved throughout evolution are shaded in black. The conserved cysteine residues and conserved prolines are shaded in grey. The position and sequences of the IB (insulin-like growth factor binding related proteins) and VWC (von willebrand type C repeat) motifs have been indicated in bold above the NOV (CCN3) consensus sequences for each domain.
- Figure 1b : Amino acid sequence alignment inn NOV (CCN3) proteins from different species has been aligned with the interalign program (University of Sandford, USA). Those residues whose position is conserved throughout evolution are shaded in black. The conserved cysteine residues and conserved pralines are shaded in grey. The position and sequences of the TSP1 (thrombospondin type 1 repeat) and CT (cystin knot) motifs have been indicated in bold above the NOV (CCN3) consensus sequences for each domain.
- Figure 2 : Distribution of sequences encoding the NOV (ccn3) gene and multimodular organisation of the NOV (CCN3) protein. (A) Schematic representation of the exon distribution in the human NOV (ccn3) gene. The sizes are given in base pairs. Boxed sequences are present in the human NOV (ccn3) cDNA clone used in previous studies (see Perbal 2001, for review). (B) Schematic representation of the five structural modules of the NOV (CCN3) protein. The signal peptide (SP) is encoded by exon 1 (el) and the domains 1-4 (D1-4) are encoded by exons 2-5 (e2-5). The length of each domain is indicated in amino acid residues. The number of cysteine residues present in each domain is shown in square brackets. The positions of amino acid sequence motifs sharing identity with insulin-like growth factor binding related proteins (IB), von Willebrand type C repeat (VWC), thrombospondin type 1 repeat (TSP1), and cystin knots (CT) are indicated below each module. The consensus sequence for each motif is indicated in figure 1.
- Figure 3 : Organisation of the S100A4 sequences in clone CLM. (A) The nucleotide sequence of the GAL4AD-CLM clone at the cloning site and (B) the protein encoded by the GAL4AD-CLM clone at the cloning site. The pGADGH vector sequences are indicated in italics. The HeLa cDNA transcripts were cloned at the EcoRI site. The nucleotide sequence contained between elb and e2 corresponds to the alternative 5' non-coding sequences characterised in human cells. The S100A4 coding sequences are contained in exons 2 and 3. The two sets of sequences in bold correspond to the calcium binding loops in the S100A4 protein. The first calcium binding site, which is encoded by exon 2, is composed of 14 amino acids with a helix-loop-helix conformation (helix A: VMVSTFHKY; loop 1: SGKEGDKFKLNKSE; helix B: LKELLTR). It corresponds to the binding loop of the pseudo EF hand (from V at position 11 to E at position 41) characteristic of the S100 proteins. The second calcium binding site, encoded by exon 3, is composed of 12 amino acids (helix C: AFQKLMSNL; loop 2: DSNRDNEVDFQE; helix D: YCVFLSC) contained in a canonical EF hand (from F at position 52 to I at position 82).

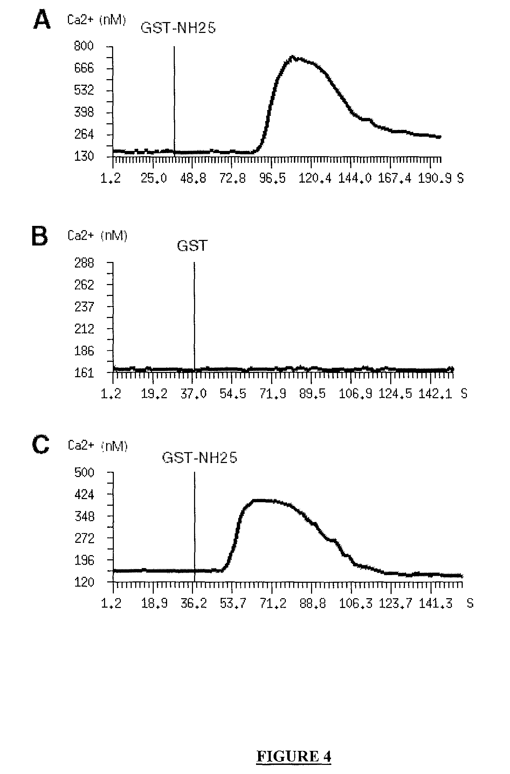

- Figure 4 : Effect of GST-NH25 on intracellular calcium increase in G59 and SK-N-SH cells. (A) The GST-NH25 protein (1 µg/ml) was added to G59 cells after 40 seconds of background recording. (B) GST only (5 µg/ml) was tested as control on G59 cells. (C) Under the same conditions, GST-NH25 (2 µg/ml) was applied to SK-N-SH cells.

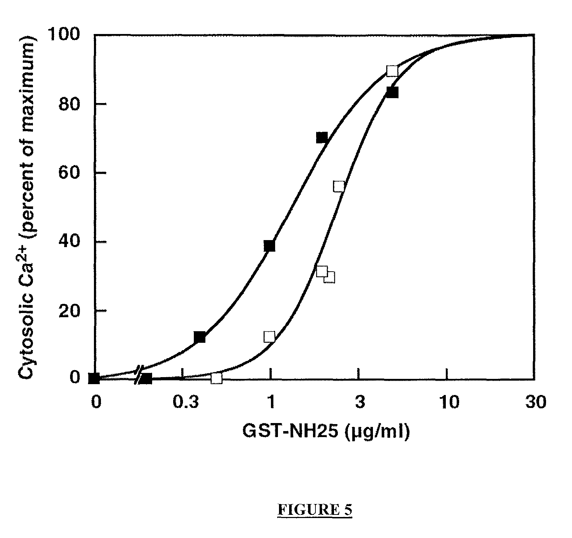

- Figure 5 : Intracellular calcium mobilisation induced by increasing concentrations of GST-NH25. Neuroblastoma SK-N-SH (open squares) or glioblastoma G59 (closed squares) cells were treated with increasing concentrations of GST-NH25 protein. The intracellular calcium influx was determined for each concentration, and represented as the percentage of the maximal activation. All other details are as described under the materials and methods section.

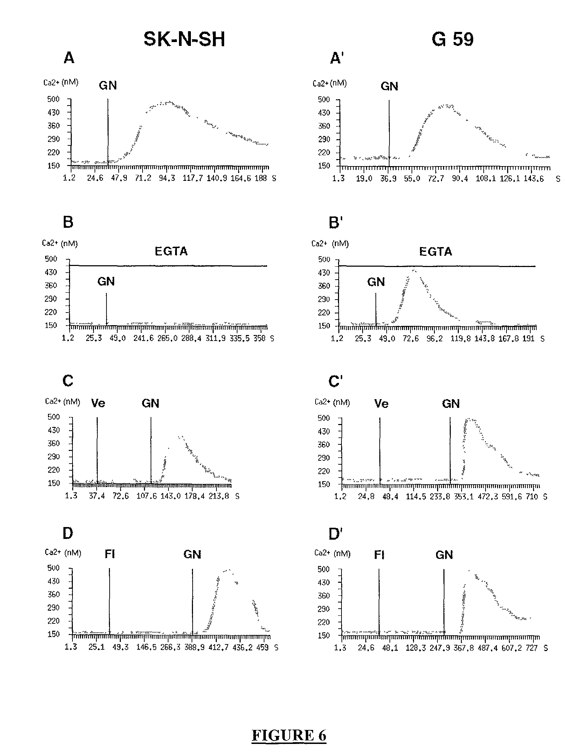

- Figure 6 : Effects of calcium blockers on intracellular calcium mobilisation induced by GST-NH25 in (A-D) SK-N-SH and (A'-D') G59 cells. GST-NH25 (2 µg/ml) was applied in (A,A') the absence or the presence of (B,B') 20mM EGTA, (C,C') 1µM verapamil, or (D,D') 10µM flunarizine.

### Concerning sodium flux :

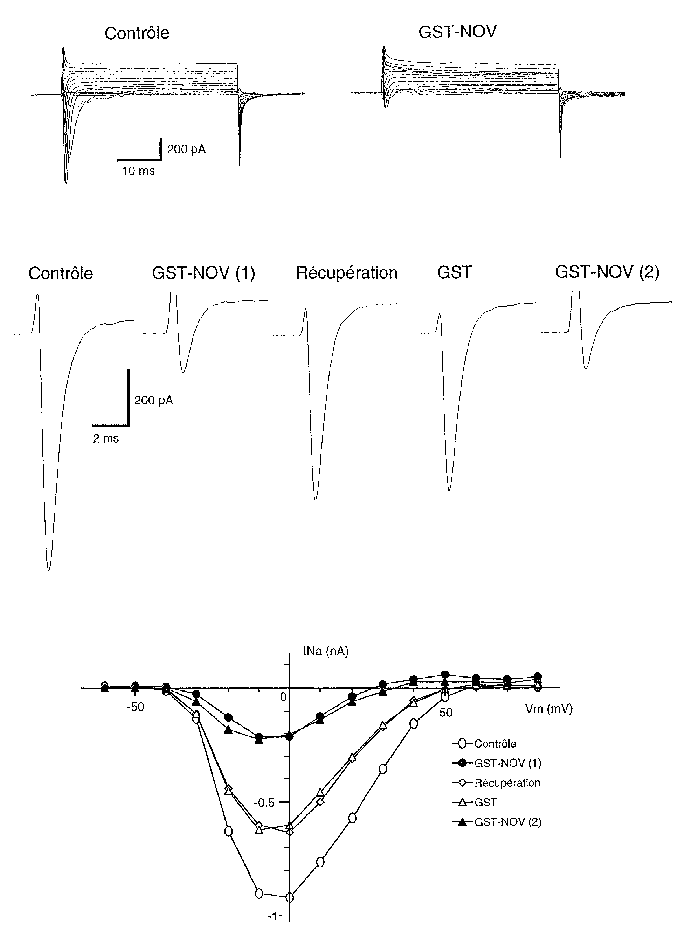

- Figures 7a to 7h : patch clamp assays measuring the sodic current in absence or presence of proteic solutions.
   The movement of ions through membranes channels constitutes the basis for many fundamental biological processes including electrical conduction along the nerves and muscle contraction.
   To measure the movement of ions through channels in the membrane of SH-NS-H cells, a blunt-tipped glass pipet was pressed gently against the cellular membrane, to form a stable physical high resistance electrical seal (commonly designated as Giga seal, in the Giga Ohm range). During patch clamping, the electrode is sealed to the cell membrane. This is done by sucking. Once good contact is made, it is possible to record ion channels opening and closing. Thus, a small patch of membrane is electrically isolated and currents going through can be measured.
   Upon the application of a 60 millivolt voltage pulse (10 ms), opening of the sodium chanels occurs and ionic movement, through these chanels, is visualized by the recording of a 900 pA current which progressively decrease to the zero values as the chanels closes (recording of the traces in the control conditions, without added protein is given on the top left panel).
   Typically, the control external Cs+-Ringer solution contained: NaCl (107 mM); Hepes (10 mM); CsCl (20 mM); NaHCO3 (4 mM); NaH2PO4 (0.8 mM); MgCl2 (1,8 mM); CaCl2 (1.8 mM); D-glucose (5 mM); sodium pyruvate (5mM); pH 7.4 buffered with NaOH.
   For measuring the effect of NOV on ion channels, the solution of GST-fusion protein was applied to the cell membrane through the pipet. The patch electrodes were filled with control internal solution which containing 10 to 50 nM of GST-NOV fusion protein, or GST protein alone (as a negative control).
   As shown on the recordings in the top right panel, addition of GST-NOV protein inhibited the sodium current. In other words, GST-NOV was interfering with sodium channel opening upon voltage pulse.
   The middle part of the figure show enlargements of the recordings performed upon voltage pulse, from left to right:
   - in the presence of control solution without added protein
   - in the presence of GST-NOV protein (inducing inhibition of sodium ion movement)
   - after washing with control solution (a partial inhibition still remains visible, suggesting that the effect of GST NOV was not totally reversed)
   - in the presence of GST protein alone, prepared in the same conditions as the GST-NOV fusion protein (no ihibitory effect, indicating that the effect was due to the NOV moiety of the fusion protein)
   - in the presence of GST-NOV (the same ihibitory effect is again observed, indicating that the sodium channels were still responding to a new delivery of NOV)
   The bottom part of the figure shows the recordings on a quantitative scale. In the control experiment , the 60 mV pulse generated a 900 pA current resulting from sodium channel(s) opening. In the presence of GST-NOV, the current was reduced to 200 pA after one or two applications. After the first application, the baseline was not back to 900 pA, but remained at 600pA indicating a partial irreversible affect on the channels (either total inhibition of a subclass of channels, or partial inhibition of all types of channels). In the presence of GST alone, the current was also measured as 600 pA, indicating that the GST alone was deproved of any inhibitory effect.

### EXAMPLES

### 1. MATERIAL AND METHODS

### Molecular Cloning

General procedures for molecular cloning were as previously described (Perbal *et al.,* 1988). The NOV (CCN3) coding sequence was amplified by using the NH2-5 and NH 5-3 primers and cloned in frame with the GST gene at the EcoR1 and SalI sites of the pGEX4T1 vector (Amersham/Pharmacia). Sequencing of the resulting clone (GST-N1125) was performed to ensure that it indeed encodes a full length native NOV (CCN3) protein fused to the GST moiety from the pGEX vector.

For constructing pGEX-CTGF, the pGEX-4T1 vector previously digested with BamH1 and SalI was first ligated with a mixture of pGEXN5 and pGEXN3 (boiled for 10 minutes and chilled in a heating bloc to 25°C) in the presence of T4 DNA ligase. This step resulted in the destruction of the original pGEX BamHI site, and introduction of a NcoI and BamHI sites. The Nco I site allowed in frame insertion of the CTGF coding sequence contained in the NcoI-BamHI CTGF DNA fragment from pACT2-CTGF (Perbal *et al.,* 1999).

Preparations of recombinant DNA were performed with the Qiagen kits. After performing the optional washing of the columns, the recombinant plasmid DNAs from minipreparations were usually eluted with 50 µl of sterile distilled water. For midipreparations, the final DNA pellet was resuspended in 300 µl of sterile distilled water.

Electro-competent cells were prepared by seeding one liter of LB medium with 10 ml of an overnight culture of the appropriate bacterial strain. The cells were grown with shaking at 250 rpm at 37°C until the culture reached an optical density of 0.7 and were then collected by centrifugation at 4000 g in a pre-chilled GS3 Sorvall rotor for 15 minutes. The bacterial pellet was resuspended in 1 liter of ice-cold sterile 10% glycerol and the suspension centrifuged at 4000 g in the GS3 Sorvall rotor for 15 minutes at 4°C. The pellet was then resuspended in 0.5 litter of ice-cold sterile 10% glycerol and centrifuged again at 4000 g for 15 minutes at 4°C. The pellet was resuspended in 20 ml of ice-cold sterile 10% glycerol, centrifuged again. The final pellet was resuspended in 2ml of ice-cold sterile 10% glycerol and the resulting preparation of electrocompetent cells was distributed in 100 µl aliquots and frozen at 80°C. The competence of the bacteria preparations was checked by performing a transformation (see below) and counting the colonies obtained with a known amount of pUC 18 DNA.

### Bacterial strains

For expression of GST fusion proteins, BL21 bacteria *(E. coli B F- ompT hsdS(rB- mB-) dcm+ Tetr gal (DE3) endA Hte)* were used. HB101 bacteria *[F-supE44 aral4 galK2 lacY*1 *leuB6 thi-1 δ(gpt-proA)62 rpsL20 (Strr) xyl-5 mtl-1 recAl3 δ(mcrC-mrr) HsdS 20 (r- m-)]* were used to recover from transformed yeast the plasmid DNA encoding the GAL4TA domain fused to target protein selected in the two hybrid screening. Cloning and amplification of other plasmids were currently performed in DH5α *[F'phi80dlacZ delta(lacZYA-argF)U169 deoR recAl endAl hsdR17 (rk-, m k+) phoA supE44 lambda-thi-1 gyrA96 relAl*/*F' proAB+ lacIqZdeltaM15 Tn10(tetr)]*.

### Transformation

Ligations of vectors and inserts to be cloned were usually performed for 18 hours at 12 °C in 50 µl reaction samples containing 10 nM ends of each DNA fragments and 1 µl of high concentration T4 DNA ligase (New England Biolabs, 400 units/µl). For transformations, ligation mixtures were mixed with 0.5 ml of electrocompetent cells in electroporation cuvettes (Biorad) on ice. Electroporation was performed with Biorad Gene pulser (Capacitance 25 µF, Resistance 300 Ohms, and 1.8 K volts). Electroporated bacteria were resuspended in 1 ml of chilled LB medium, performed with Biorad Gene pulser (Capacitance 25 µF, Resistance 300 Ohms, and 1.8 K volts). Electroporated bacteria were resuspended in 1 ml of chilled LB medium, prior to 1 hour incubation with agitation (250rpm) at 37°C and spreading on 12% agar plates containing 100 µg/ml ampicillin.

### Two hybrid system screening.

Conditions for screening the two hybrid system libraries were previously described (Perbal *et al.* Mol Pathol 1999; 52:84-91, Perbal *et al.* 1999 Proc Natl Acad Sci USA). Positive clones were restreaked twice on selective medium deprived of histidine, before checking interactions with the β-galactosidase assay. Recombinant plasmids encoding the proteins interacting with NOV (CCN3) were purified from 4 ml yeasts grown overnight in minimal medium. Cells were centrifuged in microfuge tubes and resuspended in 100 µl lysis buffer (2%Triton X100, 1% SDS, 100 mM NaCl, 10 mM Tris HCl pH 8.0, 1 mM EDTA pH 7.5), 100 µl of sterile glass beads, and 100 µl of a phenol :chloroform/isoamyl alcool mixture (49,5 :49,5 :1). After 2 minutes vortexing, the mixture was centrifuged for 20 minutes at 25°C and the upper phase used as a source of DNA. For sequencing the plasmid DNA was purified on Quiaquick columns. Plasmid DNA was usually recovered by transforming competent HB101 bacteria and selecting transformants on a minimum medium deprived of leucine and containing ampicillin.

### Production and purification of GST fusion proteins

A 20 ml sample of an overnight culture of recombinant bacteria grown in LB culture medium containing 100 µg/ml ampicillin, was used to seed one litter of ampicillin LB medium. The culture was incubated at 37°C with agitation at 250 rpm until an optical density of 0.6 was reached. At that time, IPTG was added to a final concentration of 0,1 M to induce the production of the GST-fusion protein, for 90 minutes at 37°C with agitation. Bacteria were collected after 10 minutes centrifugation at 5000 rpm in a Sorvall GSA rotor at 4°C and resuspended in 50 ml lysis buffer (10 mM Tris HCl pH 7.5, 100 mM KCl, 1 mM EDTA, 5 mM DTT, 0.5% NP40), containing 100 mM PMSF, 10 mM TPCK, 10 mM TLCK. After adding 5 ml of lysosyme solution (10 mg/ml in 25 mM Tris HCl pH 8.0) the mixture was sonicated until cells were completely broken and centrifuged for 20 minutes at 12 000 rpm in a Sorvall SS34 rotor to eliminate the cell debris. Each of the two 25 ml-fraction of supernatant was mixed with 1.5 ml of GST Sepharose beads (50% slurry in PBS), and incubated overnight at 4°C on a rotating wheel.

The GST beads were centrifuged at low speed in a table top centrifuge and washed 5 times with 6 ml binding buffer (20 mM Tris HCl pH 7.5, 100 mM KCl, 2 mM CaCl₂, 2 mM Mg Cl₂, 5 mM DTT, 0.5% NP40), containing 100 mM PMSF, 10 mM TPCK, 10 mM TLCK. Elution of the GST fusion protein was performed by resuspension in 3 ml elution buffer (10 mM reduced glutathione in 50 mM Tris HCl pH 8.0). After 1 hour agitation on a rotary wheel at 4°C, the mixture was resuspended and the supernatant collected. The elution was repeated 4 times and the different fractions kept separately. The quantity of fusion protein recovered was estimated by staining and comparison with a bovine serum albumin calibrated standard after electrophoresis on a denaturing 12% polyacrylamide gel.

The protein fractions were lyophilized and kept at -80°C. Prior use, the lyophilized fractions were resuspended in Tris HCl pH 7.5 and dialysed against the same buffer.

Control GST protein expressed by the pGEX-4T1 vector alone was produced, purified, stored and used in the same conditions.

### SDS PAGE

Polyacrylamide gel electrophoresis was performed as previously described. Except that N,N'-diallyltartardiamide (DATD, Biorad) was used instead of bisacrylamide to prepare both the separation and stacking gels.

### Primers

T7AD : 5'TAA TAC GAC TCA CTA TAG GGA GAC CAC CAC ATG GAT GAT
GTA TAT AAC TAT CTA TTC 3' (Seq ID N°1)

4ADREV2 : 5'CAG TGA GCG CGC GTA ATA CGA CTC ACT ATA 3' (Seq ID
N°2)

NH2-5 : 5'TATT GAA TTC GTC GCT GCG ACT CAG CGC TGC 3' (Seq ID N°3)

NH5-3 : 5'CTGC GTC GAC TTA CAT TTT CCC TCT GGT AGT 3' (Seq ID N°4)

pGEXN5 : 5' GATCTGGCTCTTCCAACGAATTCACCATGGGCCCGGATCCG 3'
(Seq ID N°5)

pGEXN3 : 5' TCGACGGATCCGGGCCCATGGTGAATTCGTTGGAAGAGCCA
3' (Seq ID N°6).

After synthesis, the oligonucleotides were deprotected by overnight incubation at 55°C, dried in 100 µl fractions with a speedvac with heating and resuspended in 100 µl of TE (10 mM Tris HCl pH 8.0, 1 mM EDTA) prior to loading on 1ml Sephadex G25 columns previously prepared in disposable plastic seringes. The flow through contained the purified oligonucleotides.

### PCR

For amplification of DNA fragments, 0.5 µl of the DNA template (plasmid minipreparation DNA) was mixed withl µl of both 5'-and 3'-specific primers, 2,5 µl of 10 mM dNTPs, 5 µl of 25 mM MgCl₂, 10 µl of 10X Taq buffer, 5 µl of DMSO, 74 µl of Taq polymerase, and 74 µl of sterile distilled water. PCR was performed for 35 cycles (each cycle being 95°C for 2 minutes, 50°C for 3 minutes and 72°C for 3 minutes). The PCR products were run in 1,2 % agarose gel in TAE buffer and eluted with the Qiagen PCR purification kit, under conditions recommended by the supplier.

The templates for *in vitro* transcription/translation were prepared as follows. 0.5 µl of each minipreparation DNA plasmid was mixed with 10 µl buffer, 4.8 µl MgCl₂, 10 µl DMSO and 62.2 µl water, as described above. The mixture was boiled for 5 minutes and chilled in ice before addition of 7.5 µl water, 2.0 µl 10 mM dNTPs and 0.5 µl of Taq polymerase and incubation for 35 cycles of 95°C for 1 minute, 60°C for 1 minute, 72°C for 1 minute.

### In vitro transcription/translation

The *in vitro* transcription/translation of PCR amplified templates was performed with the TNT Lysate reaction mix (Promega) as recommended by the supplier, with the following modifications :

PCR products were extracted once with 200 µl of a 24 :1 mixture of chloroform : isoamyl alcool, and precipitated by addition of 15 µl 3M sodium acetate 500 µl of pre chilled (-20°C) absolute ethanol. After 10 minutes incubation in a dry ice-ethanol bath, the DNA precipitate was collected by 10 minutes centrifugation at 12000 rpm in a microcentrifuge, washed once with 70 % chilled ethanol and dried by speed-vac centrifugation at medium temperature. The DNA precipitate was resuspended in 6 µl of sterile distilled water and mixed with 25 µl of the TNT reticulocyte extract, 2 µl of the TNT reaction buffer, 1 µl of T7 RNA polymerase, 1 µl of 1 mM amino acid mixture deprived of methionine, 1 µl of RNAsin ribonuclease inhibitor, and 4 µl of ³⁵ S methionine (37 TBq/mmol, 10 mCi/ml). The mixture was incubated for 1H30 at 30°C and the *in vitro* proteins analyzed by electrophoresis in a 15% polyacrylamide gel and autoradiography.

### Sequencing

DNA sequencing was performed routinely at Genomex (Grenoble, France).

### GST Pull down

Twenty microliter samples of ³⁵S labelled protein from IVT were incubated for 18 hours at 4°C on a rotating wheel with 5 µl of GST-nov sepharose beads in a total reaction volume of 100 µl of binding buffer (20 mM Tris HCl pH 7.5, 100 mM KCl, 2 mM CaCl₂, 2 mM MgCl₂, 5 mM DTT, 0.5% NP40, 5% Glycerol, 100 mM PMSF , 10 mM TPCK, 10mM TLCK). After incubation, the GST sepharose beads were washed five times with 2 ml of chilled PBS and the protein complexes bound to the beads were analyzed by electrophoresis on a 15 % polyacrylamide gel and autoradiography.

### Cell cultures

The glioblastoma G59 cells have been described previously (Li WC *et al.* 1996 Mol Pathol). The SK-N-SH are neuroblastoma-derived cells. Cells were grown in a 5% CO₂ atmosphere at 37°C in DMEM supplemented with 10% serum (for G59) or 5% serum (for SK-N-SH), 2 mM glutamate, 200 µg/ml penicillin and 100µg/ml streptomycin sulphate.

### Intracellular calcium measurements

The transient increase of intracellular calcium concentration was measured in response to activation of SK-N-SH and G59 cells using a dynamic imaging microscopy system QuantiCell 700 (VisiTech international Ltd., UK) with 15-20 cells per field as described earlier (Banisadr G *et al.* 2000 ; Yamada M *et al.* 1998). Cells were grown on glass coverslips (22 mm) for 4 days. They were then loaded with 4 mM Fura-2 AM (Molecular Probes, Interbiotech, France) in PBS supplemented with 1.3 mM CaCl₂, 0.8 mM MgCl₂, 5 mM glucose and 20 mM Hepes-Tris (PBSc), pH 7.4 at 37°C for 1 h. After washing with PBSc and background recording for 40 sec (20 images), the cells were treated with GST-NH 25 or GST alone as control. Fluorescence images were recorded every 2 seconds and intracellular calcium concentration was calculated from the ratio of the fluorescence intensities at 340 and 380 nm on a pixel basis. The dose-response curve for intracellular calcium concentrations was obtained by integrating the area under the curve (measuring Ca ²⁺ transients plotted as a function of time for each field from the addition of the GST-NH25 until the end of image recording, 200 sec) and averaging the fluorescence from the whole field of cells chosen (Mazor *et al.* 2002). The Ca 2⁺ stimulation curves were determined for each tested concentration of GST-NH25.

### 2. RESULTS

### 2.1 NOV (CCN3) physically interacts with S100A4 calcium binding protein.

A HeLa cell cDNA library fused with sequences encoding the yeast Gal4 transcriptional activation domain in the pGADGH vector, was used to screen for proteins interacting with NOV (CCN3). The NOV (CCN3) pGBT9-derived bait plasmid was obtained after fusing the NOV (CCN3) sequences encoding domains 2-4 (figure 2) in frame with the DNA binding domain of Gal4 (Perbal *et al.* Mol Pathol 1999; 52:84-91, Perbal *et al. 1999* Proc Natl Acad Sci USA). The rationale for using the coding sequence NOV (CCN3) of the full length secreted NOV (CCN3) protein was based on the assumption that specific interactions might involve structural motifs resulting from the tertiary structure of the NOV (CCN3) protein and from physical interaction of different domains. The screening was performed as described in material and methods and permitted to isolate several clones encoding proteins interacting with NOV (CCN3). In order to check that candidate clones were encoding proteins that were indeed able to physically interact with NOV (CCN3) outside of the yeast nucleus, the plasmids encoding the potential targets were purified after transformation of HB101 bacteria and PCR-amplified before being used for *in vitro* transcription/translation (see material and methods). The ³⁵S labelled proteins synthesized from the PCR amplified templates were used to run GST pull down assays. In many cases, binding of *in vitro* labelled proteins to GST-NOV (GST-CCN3) sepharose beads took place as shown by SDS-PAGE. The sequences of the corresponding cloned inserts were established and analysed with the BLAST program from NCBI [(National Center for Biotechnolgy Information (http://www.ncbi.nlm.nih.gov)].

Among the different plasmids that were encoding potential partners of NOV (CCN3), four independent clones shared a high degree of identity with the S100A4 calcium binding protein [also designated p9Ka, calvasculin, CAPL, mtsl, pEL98, 18A2, 42A and fsp (for a review see ref Barraclough R *et al.* 1998). The longest clone showed 100% identity with S100A4 mRNA over 512 nucleotides. Analysis of the insert sequences indicated that it encoded the full length 101 aminoacid residues long S100A4 protein (figure 3). Interestingly, the 5' non coding sequences localized upstream to the S100 A4 protein iniation codon, were inserted in frame with both the Gal4 transactivation domain of pGAD-GH and S100A4, thereby encoding a stretch of 25 aminoacid residues inserted between the two fused proteins. The 5' proximal sequences of the S100A4 insert corresponded to an alternative non coding exon designated elb, which was first identified in S100A4 cDNA species isolated from a human osteosarcoma library and reported to be more abundantly expressed in HeLa cells and human adrenal carcinoma cells (TCAR), and to be predominant in lung carcinoma.

The interaction of NOV (CCN3) with S100A4 raised the possibility that it might also be involved at some stage of calcium signalling and led us to check whether NOV (CCN3) itself might exhibit any effect on the intracellular calcium levels.

### 2.2 Induction of intracellular calcium waves by CCN3.

The application of GST-NH25 resulted in a transient increase of the intracellular calcium level in G59 glioblastoma cells (figure 4A). When the control GST protein alone was applied, no modification of the level of intracellular calcium was observed (figure 4B). A similar calcium transient increase was also observed with human neuroblastoma cells SK-N-SH upon GST-NH25 delivery (figure 4C).

Furthermore, a transient stimulation of the increase of intracellular calcium was also induced when GST-CCN2 was applied on G59 cells (data not shown). No increase was induced by the GST-fibulin 1C fusion protein which contained the portion of fibulin 1C interacting with NOV (CCN3) (Perbal *et al.* 1999 Proc Natl Acad Sci USA).

The calcium response was dependent upon GST-NH25 concentrations. The amplitude of calcium transient waves increased with the amount of GST-NH25 added, to reach a maximal level at 10 µg/ml of GST-NOV (GST-CCN3) fusion protein. Toxic effects (up to cells blowing up) were observed when higher concentrations of GST fusion protein were used. The dose-response curves presented in figure 5 showed IC50 values of 1.3 and 2.4 µg/ml of GST-NH25 in G59 and SK-N-SH cells respectively.

To determine the origin of this calcium transient increase, different calcium blockers were tested (figure 6) on both SK-N-SH (A-D) and G59 (A'-D') cell lines. The intracellular calcium level increased after application of 2 µg/ml of GST-NH25 (GN) on SK-N-SH (figure 6A) but this increase was totally inhibited in a calcium depleted medium (20 mM EGTA) (figure 6B). Conversely, in the same conditions, GST-NH25 induced a calcium transient increase in the G59 cells, even in the absence of extracellular calcium (figure 6B').

Furthermore, the addition of CaCl₂ to EGTA-containing medium, induced a sustained increase of intracellular calcium level, giving strong support to a mixed interaction of NOV (CCN3) with both stimulation and release of intracellular calcium from the internal stock and with the entry of extracellular calcium in G59 cells.

The addition of verapamil (1 µM; figure 6C, 6C'), a blocker of voltage-dependant calcium channels (Spedding *et al.* 1992) or the addition of flunarizine (10 µM; figure 6D, 6D'), a blocker of both voltage-dependant sodium and calcium channels (Spedding *et al.* 1992 Pharmacol Rev) did not modify the calcium transient increase induced by GST-NH25 in the two types of cell lines.

The inventor wishes to add the following comments to these results. It is now well established that cytoplasmic calcium (Ca 2⁺) is a key messenger that regulates many different biological functions including cellular communication, proliferation and differentiation. The levels of intracellular calcium are tightly controlled by processes involving both uptake from exogenous sources and mobilisation of intracellular stores associated with the endoplasmic reticulum network. The entry of calcium inside the cells is controlled by a complex set of channels that can open either upon binding of an external signalling ligand (voltage and receptor operated channels) or as a result of calcium depletion of internal stores (store operated channels). Several classes of cell surface receptors playing key roles in cell-cell and cell-extracellular matrix adhesion have been reported to be calcium binding proteins. For example, a number of integrins are known to stimulate changes in the levels of intracellular calcium associated with the regulation of cell fate and with development (Sjaastad *et al.* 1996 ; Sjaastad *et al.* 1997).

In many cells, biological regulators induce propagating and oscillatory rises in intracellular concentration as a result of calcium release form the endoplasmic reticulum. The spatiotemporal regulation of calcium release involves several channels of which the inositol 1,4,5-triphosphate (IP3) receptors have been the most extensively studied, with the Ryanodine receptors (RyR) (for recent reviews see Cullen *et al.* 2002 ; Bootman *et al.* 2001). The flow of calcium which enters the cell cytoplasm could be deleterious if the levels of intracellular calcium were not controlled by buffer systems which regulate the delivery of calcium in the cell. The S100 family of proteins are among those playing a role of calcium sensor and buffer.

The S100A4 protein is a 101 amino acid calcium binding protein. It belongs to the S100 (soluble in 100 % ammonium sulfate) family of proteins whose first members were isolated from bovine brain (Moore *et al.* 1965). To date 16 members sharing various degrees of amino acid sequence identity compose the S100 family of proteins. They contain two calcium binding sites with an helix-loop-helix structure (EF-hands). As it is the case in other S100 proteins, the N-terminal EF-hand site of S100A4 is composed of a 14-residue calcium binding loop with a high number of basic residues, whereas the C-terminal EF-hand of S100A4 encompass a canonical 12 amino acids loop including several acid residues (figure 3) (for reviews see Smith *et al.* 1998 ; Nelson *et al.* 1998). The two EF-hands motifs display different affinities for the calcium and the S100 proteins are generally thought to modulate the propagation of calcium signals via their ability to bind calcium.

Another aspect of the relationship existing between NOV (CCN3) function and calcium signalling was brought up by the inventor by the marked increase of intracellular calcium concentration which was transiently induced in NOV-treated G59 and SK-N-SH cells.

The results obtained with blockers of voltage-dependent calcium and sodium channels allowed to distinguish between two levels of action for NOV (CCN3). In the case of G59 cells both entry and mobilisation of internal calcium stock were induced by the addition of NOV (CCN3) whereas only entry of external calcium was induced in the case of SK-N-SH cells.

Extracellular uptake of calcium induced by NOV (CCN3) was shown by the inventor to proceed even when voltage-dependent channels were blocked. Therefore, it involves store-operated calcium channels (SOCs), a heterogeneous subset of plasma membrane C^{a2+} channels. The best characterized channel of this type was originally described in mast cells in which depletion of calcium induced a sustained calcium inward current that was not voltage-activated and therefore termed ICRAC (for calcium release-activated calcium) (Parekh *et al.* 1997 ; Hoth *et al.* 1992).

Internal mobilisation of intracellular calcium stores by NOV (CCN3) in G59 cells might involve specific receptors and IP3-activated channels.

The lack of response of SK-N-SH cells to GST-NH25 in the absence of an extracellular pool of calcium might result from differences in the receptors of these cells, or from biochemical variations in the treatment of information resulting form the presence of an inhibitor or the lack of a transmitter in SK-N-SH cells.

### 2.3 Action of NOV protein on sodium flux.

The NOV protein eluted from a glutathione sepharose column (sigma) was lyophilised and re-dissolved in PBS (phosphate buffer saline) for use.

The experiments of patch clamp were made according to standard protocols, with concentration of purified recombinant proteins purified ranging from 10 to 50 nM. The quantity and quality of the proteins preparations used were evaluated by electrophoresis on polyacrylamide gel in denaturing medium ("A Practical Guide to Molecular Cloning". 2^{nd} Edition. Wiley & Sons, New York).

The cells used were neuronal cells SK-NS-H as described above. The neuronal SK-NS-H cells (ATTCC HTB-11) were provided by Dr. A. Lombet, and were grown in MZM Eagle containing 2 mM glutamine, 1.5 g/l sodium carbonate, 0.1 mM non essential amino acids, 0.1 mM sodium pyruvate, 10% fetal bovine serum (Gibco). Cells were passaged by trypsinization with 0.25% trypsin, 00.3% EDTA, before resuspension in fresh culture medium containing fetal calf serum. Cells were rinsed with control solution before performing the patch clamp experiments. These cells were cultivated according to standard protocols.

The patch clamp technique measures the sodic current in absence and in presence of the proteic solutions. The figure 7 shows :
a/ The control situation (-900 pA) in absence of any protein.
b/ The current measured after adding of a solution of fusion protein GST-NOV (-20 pA).
c/ The current measured after washing (-600 pA) : a partial recuperation is obtained, that can be due to a change of intervening channels.
d/ The current measured after adding a solution of GST only, prepared according to the same conditions as the fusion protein (-600 pA).
e/ The current measured after a second adding of the solution of the protein fusion GST-NOV (-20 pA) : this last value shows that the mechanism is irreversible, the channel is not blocked definitively after the first adding.

Thus, the results obtained by the inventor demonstrate that protein NOV used is capable of blocking the sodic current. These results are very interesting since they are comparable to those obtained with the tetrodoxin compounds mentioned above. Besides the NOV protein used is a stable protein (the stability of NOV in culture medium has been shown by Chevalier et al.), and has a reversible effect, allowing local use or remote use that can not be obtained with diffusible chemical compounds.

### REFERENCES

Ayer-Lelievre C, Brigstock D, Lau L, et al. Report on the first international workshop on the CCN family of genes. Mol Pathol 2001;54:105-20.
Banisadr G, Dicou E, Berbar T, et al. Characterization and visualization of [125I] stromal cell-derived factor-1 alpha binding to CXCR4 receptors in rat brain and human neuroblastoma cells. J Neuroimmunol 2000; 10:151-60.
Barraclough R. Calcium-binding protein S100A4 in health and disease. Biochim Biophys Acta 1998;1448:190-9.
Berridge M, Lipp P, Bootman MD. The versatility and universality of calcium signaling. Nat Rev 2000; 1:11-21.
Bootman MD, Lipp P, Berridge MJ. The organisation and functions of local CA2+ signals. J Cell Sci 2001;114:2213-22.
Bork P. The modular architecture of a new family of growth regulators related to connective tissue growth factor. FEBS Lett 1993;327:125-30.
Chevalier G, Yeger H, Martinerie C, et al. NovH: differential expression in developing kidney and Wilms' tumors. Am J Pathol 1998;152:1563-75.
Cullen PJ, Lockyer PJ. Integration of calcium and Ras signaling. Nat Rev 2002;3:339-48.
Glukhova L, Angevin E, Lavialle C, et al. Patterns of specific genomic alterations associated with poor prognosis in high-grade renal cell carcinomas. Cancer Genet Cytogenet 2001;130:105-10.
Gupta N. et al. inhibition of glioma cell growth and tumorigenic potential by CCN3 (NOV). Mol Pathol 54:293-299.
Hashimoto BY, Shindo-Okada N, Tani M, et al. Expression of the ELMI gene, a novel gene of the CCN (connective tissue growth factor, Cyr61/Cef10 and neuroblastoma overexpressed gene) family, suppresses in vivo tumor growth and metastasis of K-1735 murine melanoma cells. J Exp Med1998;187:289-96.
Hoth M, Penner R. Depletion of intracellular calcium stores activates a calcium current in mast cells. Nature 1992;355:353-6.
Joliot V, Martinerie C, Dambrine G, et al. Proviral rearrangements and overexpression of a new cellular gene (nov) in myeloblastosis-associated virus type 1-induced nephroblastomas. Mol Cell Biol 1992;12:10-21.
Katsube K, Chuai ML, Liu YC, et al. The expression of chicken NOV, member of the CCN family, in early stage development. Brain Research Gene Expression Patterns 2002;1:61-5.
Kiselyov K, Shin DM, Shcheynikov N, et al. Regulation of Ca2+-release-activated Ca2+ current (Icrac) by ryanodine receptors in inositol 1,4,5-trisphosphate-receptor-deficient DT40 cells. Biochem J 2001;360:17-22.
Kocialkowski S, Yeger HJ, Kingdom C, et al. Expression of the human NOV gene in first trimester fetal tissues. Anat Embryol 2001;203:417-27.
   expression in human central nervous system. C R Acad Sci III 1998;321:883-92.
Kumar S, Hand AT, Connor JR, et al. Identification and cloning of a connective tissue encoding a novel regulator of osteoblast functions. J Biol Chem1999;274:17123-31.
Lau LF, Lam SC. The CCN family of angiogenic regulators: the connection. Exp Cell Res 1999;248:44-57. 3Brigstock DR. The connective tissue growth factor/cysteine-rich 61/nephroblastoma overexpressed (CCN) family. Endocr Rev 1999;20:189-206.
Li WC, Martinerie C, Zumkeller W, et al. Differential expression of novH and CTGF in human glioma cell lines. Mol Pathol 1996;49:91-7.
Maillard M, Cadot B, Ball RY, et al. Differential expression of novH proto-oncogene in human prostate cell lines and tissues. Mol Pathol 2001;54:275-80.
Manara MC, Perbal B, Benini S, et al. The expression of ccn3 (Nov) in musculoskeletal tumors. Am J Pathol 2002;160:849-59.
Martinerie C, Viegas-Pequignot E, Guenard I, et al. Physical mapping of human loci homologous to the chicken nov proto-oncogene. Oncogene 1992;7:2529-34.
Mazor O, Hillaret de Boisferon M, Lombet A, et al. Europium-labeled epidermal growth factor and neurotensin: novel probes for receptor-binding studies. Anal Biochem 2002;301:75-81.
Moore BW. A soluble protein characteristic of the nervous system. Biochem Biophys Res Commun1965;19:739-44.
Nelson MR, Chazin WJ. Structures of EF-hand Ca2+-binding proteins: diversity in the organization, packing and response to Ca2+ binding. Biometals 1998; 11 :297-318.
Parekh AB, Penner R. Store depletion and calcium influx. Physiol Rev 1997;77:901-30.
Pennica D, Swanson TA, Welsh JW, et al. WISP genes are members of the connective tissue growth factor family that are up-regulated in Wnt-1-transformed cells and aberrantly expressed in human colon tumors. Proc Natl Acad Sci U S A1998;95:14717-22.
Perbal B. NOV (nephroblastoma overexpressed) and the CCN family of genes: structural and functional issues. Mol Pathol 2001 ;54:57-79.
Perbal B, Martinerie C, Sainson R et al. The C-terminal domain of the regulatory protein NOVH is sufficient to promote interaction with fibulin 1C : a clue for a role of NOVH in cell-adhesion signalling. Proc Natl Acad Sci USA 1999; 96:869-74.
Perbal B. Contribution of MAV-1-induced nephroblastoma to the study of genes involved in human Wilms' tumor development. Crit Rev Oncog 1994;5:589-613.
Perbal B. Les protéines CCN: quand multimodulaire rime avec multifonctionnel. Médecine Sciences 2002; 18:583-92.
Perbal B. Nuclear localization of NOV protein: a potential role for nov in the regulation of gene expression. Mol Pathol 1999;52:84-91.
Perbal B. Caractérisation et expression du proto-oncogène nov humain dans les tumeurs de Wilms. Bull Cancer (Paris) 1994;81:957-61.
Sjaastad MD, Lewis RS, Nelson WJ. Mechanisms of integrin-mediated calcium signaling in MDCK cells: regulation of adhesion by IP3- and store-independent calcium influx. Mol Biol Cell 1996;7:1025-41.
Sjaastad MD, Nelson WJ. Integrin-mediated calcium signaling and regulation of cell adhesion by intracellular calcium. Bioessays 1997;19:47-55.
Spedding M, Paoletti R. Classification of calcium channels and the sites of action of drugs modifying channel function. Pharmacol Rev 1992;44:363-73.
Smith SP, Shaw GS. A change-in-hand mechanism for S100 signalling. Biochem Cell Biol 1998;76:324-33.
Su BY, Cai WQ, Zhang CG, et al. A developmental study of novH gene
Tanaka S, Sugimachi K, Saeki H, et al. A novel variant of WISPI lacking a Von Willebrand type C module overexpressed in scirrhous gastric carcinoma. Oncogene 2001;20:5525-32.
Thomopoulos G, Kyurkchiev S, Perbal B. Immunocytochemical localization of novH and ultrastructural characteristics of NCI-H295R cells. J Submicrosc Cytol Pathol 2001;33:251-60.
Yamada M, Lombet A, Forgez P, et al. Distinct functional characteristics of levocabastine sensitive rat neurotensin NT2 receptor expressed in Chinese hamster ovary cells. Life Sci 1998;62:375-80.
Zhang R, Averboukh L, Zhu W, et al. Identification of rCop-1, a new member of the CCN protein family, as a negative regulator for cell transformation . Mol Cell Biol 1998;18:6131-41.

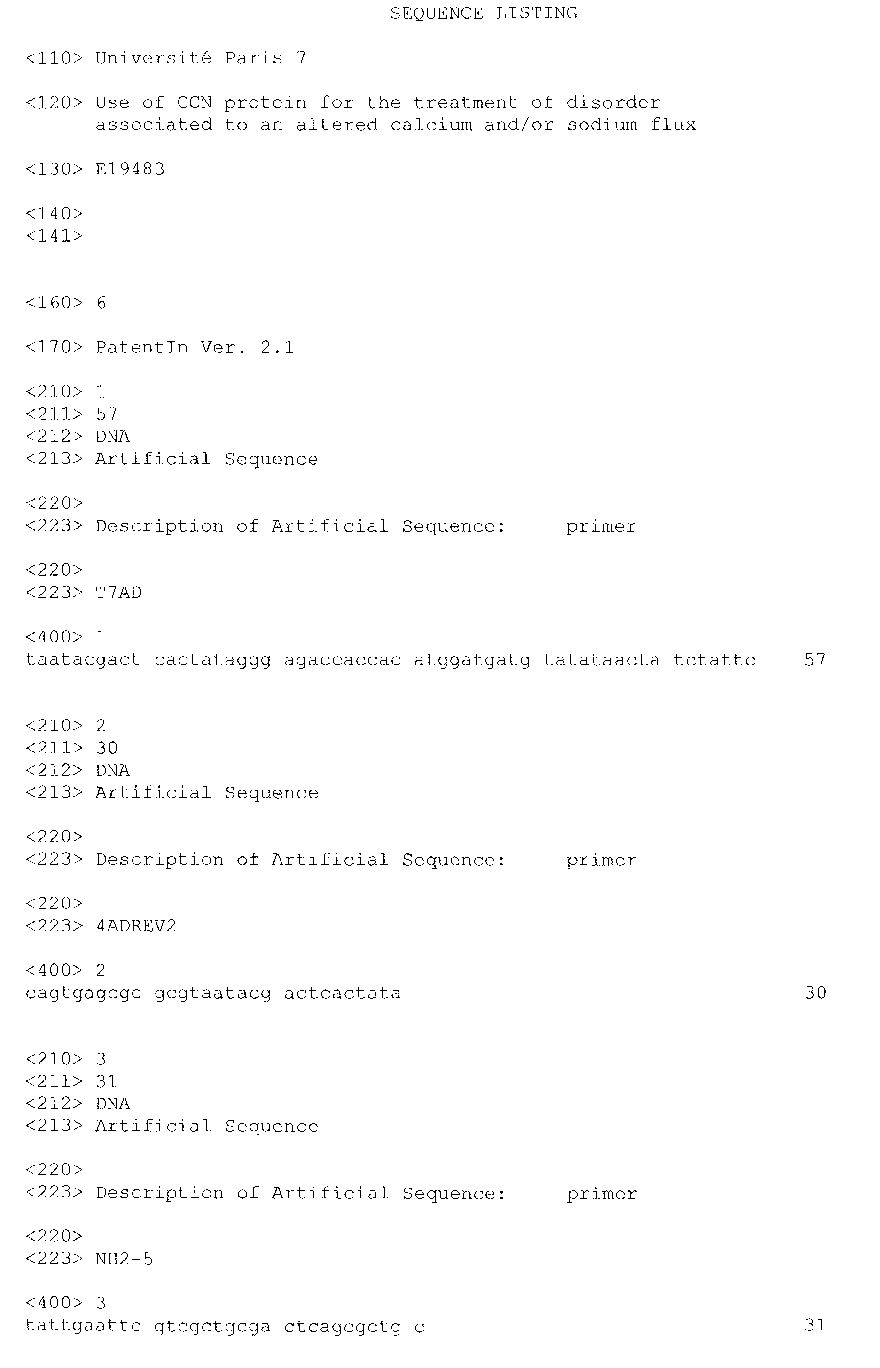

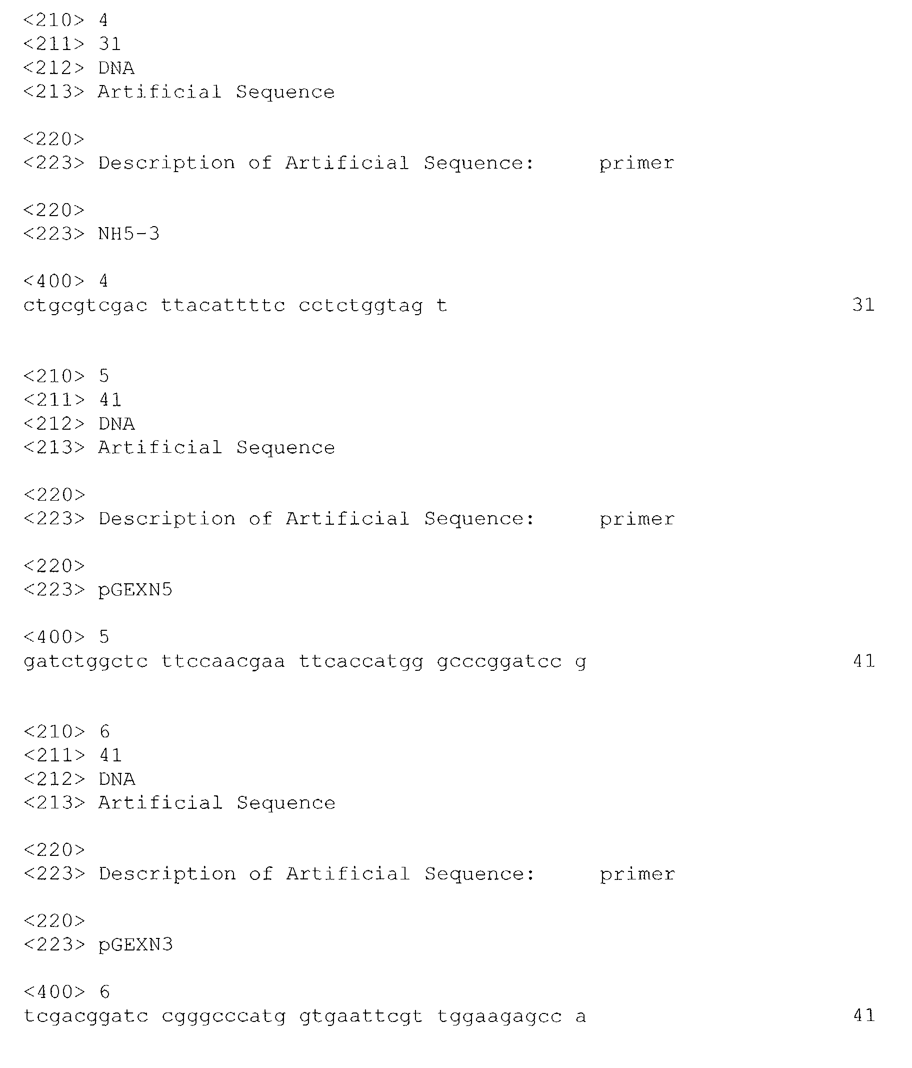

## Claims

**1.** Use of a CCN protein for the preparation of a drug for the treatment of a disorder associated to an altered calcium and/or sodium flux.

**2.** Use of a CCN protein for the preparation of a drug for the treatment of a disorder associated to an altered calcium flux.

**3.** Use of a CCN protein for the preparation of a drug for the treatment of a disorder associated to an altered sodium flux.

**4.** Use according to claim 1 to 3 wherein CCN protein is NOV protein

**5.** Use according to claim 2 wherein said disorder is a disorder chosen among cellular repair, signalisation of cellular adhesion mediated by integrins, cellular interaction and communication.

**6.** Use according to claim 2 wherein said disorder is a disorder of cardiac cells and/or skeletic muscles cells, namely a pathology of muscular contraction or a neurodegenerative disease.

**7.** Use according to claim 2 wherein said disorder is a disorder of bone growth, bone repair.

**8.** Use according to claim 3 wherein said protein presents an antalgic or analgesic activity.

**9.** Use according to claim 3 wherein said disorder is a behaviour disorder.

**10.** Use according to claim 3 wherein said disorder is associated to altered nervous conduction.

**11.** Use according to claim 3 wherein said disorder is associated to altered listening and knowing functions.

**12.** Pharmaceutical composition comprising at least one CCN protein and a pharmaceutically acceptable carrier.

**12.** Pharmaceutical composition comprising NOV protein and a pharmaceutically acceptable carrier.

**13.** Method of screening of a CCN protein efficient against a disorder associated to altered calcium flux, comprising adding an appropriate dosage of a tested CCN protein, and measuring the intracellular calcium level, eventually using NOV protein as control.

**14.** Method of screening of a CCN protein efficient against a disorder associated to altered sodium flux, comprising adding an appropriate dosage of a tested CCN protein, and measuring the intracellular sodium level, eventually using NOV protein as control.

**15.** Use of a CCN protein obtained by a method of screening of claim 13 or 14, for the treatment and/or prophylaxis of a disorder associated to altered calcium and/or sodium flux.
